# EUROPEAN PATENT APPLICATION

(11) **EP 4 332 552 A1**
(43) Date of publication of application: **06.03.2024**
(21) Application number: 23724896.8
(22) Date of filing: 26.04.2023
(51) Int. Cl.: G01N 21/64, C12Q 1/686, G01N 21/03

(54) **DEVICE FOR DETECTING INDUSTRIAL PATHOGENS**

(30) Priority: 30.08.2022 ES 202230778
(71) Applicant: Thenextpangea, S.L, 33418 Gozón - Asturias (ES)
(72) Inventor: FERNÁNDEZ MORENO, Marta, 33418 Gozón - Asturias (ES); ALICIA, Fernández Suárez, 33418 Gozón - Asturias (ES); GARCÍA OSORIO, Fernándo, 33418 Gozón - Asturias (ES); MENÉNDEZ PAREDES, Guillermo, 33418 Gozón - Asturias (ES); RICO FERNÁNDEZ, José, 33418 Gozón - Asturias (ES); SANZ MORAL, Luis Miguel, 33418 Gozón - Asturias (ES); SORIA VALLES, Clara, 33418 Gozón - Asturias (ES)
(74) Representative: Clarke, Modet y Cía., S.L.
(86) International application number: PCT/ES2023/070266
(87) International publication number: WO 2023/222933

(57) **Abstract**

Device (1) for detecting a pathogen in a sample (2). The device (1) includes a first housing (10) for reactions with a illumination source and two compartments (4). In each compartment (4) a reaction tube (12) is fixed in a position illuminated by the illumination source. Each compartment (4) includes a photodetector for analogically detecting a portion of light signal reflected by the reaction tube (12). The device has a photographic camera (13) for simultaneously capturing a digital image of the two compartments (4) with the reaction tubes (12). There is furthermore a heater (17) for heating the reaction tubes (12). With a processing means, the analog information is compared with the digital information obtained from the image of each reaction tube (12) and with an expected value corresponding to a specific pathogen that indicates its presence in the sample.

## Description

### TECHNICAL FIELD OF THE INVENTION

The invention pertains to the field of detecting industrial pathogens, more specifically, bacteria, viruses, protozoa, or other organisms capable of affecting public and/or environmental health. The invention relates to a portable and easy-to-use pathogen detection device, e.g. *Legionella* or *Listeria.*

### BACKGROUND OF THE INVENTION

Industrial pathogens are defined as those microorganisms, whose presence in an industrial human operating environment is associated with the generation of public health and/or environmental problems.

For this reason, the detection of these pathogens is the object of intense activity and regulatory control. Next, are two particularly representative examples of this definition, *Legionella* and *Listeria.*
*(1) Legionella pneumophila,* commonly referred to as legionella, is a species belonging to the genus *Legionella* bacteria. It generally resides in natural aquatic environments, but can also be established in the water supply network of buildings and in systems that require water for their operation.

The infection that causes *Legionella* is called Legionellosis. It can present as a febrile illness, either mild in character (Pontiac fever), or severe as an atypical pneumonia (Legionnaires disease). It is contracted through the airways by inhalation of sprays. For this reason, air conditioners, humidifiers, dew machines, etc. are possible sources of contagion. Recently, a significant increase in the number of cases has been observed and the authorities have reacted with legislative changes that require more frequent control of the presence of *Legionella.*

(2) The bacterium *Listeria monocytogenes* resides in different types of environments, soil, water, vegetation or even in animals. The infection in humans usually comes from food contamination, since this bacterium has the ability to survive, and even grow in refrigerated foods or treated with other preservation measures.

Infection with *Listeria monocytogenes* causes listeriosis disease, which depending on the severity can cause moderate symptoms such as fever, muscle pain or nausea, or severe symptoms such as headache, muscle stiffness, confusion, loss of balance or seizures. Listeriosis can cause death in very young people, the elderly, or people with compromised immune systems.

There are several techniques for the detection of industrial pathogens, which, roughly, can be classified into three categories:
Microbiological cultures: This is the reference technique and there are several standard tests. This technique requires extended incubation periods (up to 7 days), very specific culture means, lengthy sample preparation processes, etc.

Molecular methods (qPCR): They are somewhat faster and more sensitive techniques, allowing very low concentrations of genetic material to be detected. However, they currently require laboratories with specialized equipment and personnel.

Immunological tests: They are based on the antigen-antibody interaction, although this interaction has to be associated with visualization techniques. Depending on the visualization technique used, the specific characteristics of the detection change. In some cases, specialized laboratories and longer assay times are required, such as Enzyme-Linked Immunosorbent Assays (ELISA). In other cases, however, lateral flow strips may be used to reveal the result in a short time, albeit with less sensitivity and no ability to quantify pathogen levels.

It is noted that the current techniques have shortcomings. Mainly, they require an excessive time (sometimes, around 2 weeks), they are expensive in terms of personnel and equipment. Others offer results that are qualitative or unreliable.

### DESCRIPTION OF THE INVENTION

An object of the invention is a device according to the independent claim which is conceived in view of the problems identified. Particular embodiments of the invention are defined in the dependent claims.

A general advantage of the device is that data can be acquired in a dual manner. Two independent signals of different nature are recorded, one digital signal coming from the photographic camera, and another of analog nature coming from the photodetector, later also converted into a digital signal.

Another advantage of certain embodiments of the device is that it allows for two modes of operation. In the first one, digital and analog images are acquired with identical optical filters for two compartments one with the sample, the other one with sterile substance. In this first mode of operation, detection accuracy and robustness are maximized. Both signals are processed independently, so that only when both are signals are indicative of positive detection, the end result of the analysis shown by the device will be positive. Alternatively, in the second mode of operation of the device two independent signals are detected in each compartment. Each signal can be operated independently, by using a certain optical filter for the detection reaction of each pathogen, and allowing the simultaneous detection of two different pathogens.

### BRIEF DESCRIPTION OF THE DRAWINGS

Embodiments of the present invention are described, by way of example only, in the accompanying drawings in which like elements are numbered in the same way in the various Figs.:
**Fig. 1A** is a representation of the external appearance of the analysis device. **Figs. 1B-1C** is a structural schematic representation of the device. **Fig. 1B** exploded. **Fig. 1C** inside.
**Fig. 2A** Detailed plane of the housing for perspective device reactions. **Fig. 2B** in plant. **Fig. 2C** in section.
**Fig. 3** Schematic representation of several stages of the procedure for detecting a pathogen in a sample related to the preparation.
**Fig. 4** Schematic representation of several stages of the procedure of detecting a pathogen in a sample related to information acquisition and analysis.
**Fig. 5** Representation of the graphical interface of the device.

### Glossary of numerical references:

1 Device.
2 Top lid.
3 Touch-screen.
4 Sample compartment.
5 Switch.
6 Lower lid.
7 Frame.
8 Connection for power supply.
9 Sample receptacle.
10 First housing (for reactions).
11 Second housing (for electronics).
11A Processor.
12 Reaction tube.
13 Photographic camera.
14a, 14b Acrylic filters.
15 LED, Light-emitting diode.
16 Photodiode.
17 Heater.
18 Amplifier.
19 Separating wall.
20 Protective lens cover.
21 Sample pre-processing stage.
22 Reaction stage.
23 Data acquisition stage
24 Data pre-processing stage
25 Prediction stage
26 Visualization stage
26a Start/Stop Button
26b Device status
26c Experiment progress bar
26d Visualization of measurements
26e Export results button
26f Measurement result
31 Mixing step
32 Lysis step
33 Step of placing the sample
34 Step of placing a sterile substance
35 Step of analog information collection
36 Step of digital information collection
37 Step of analogue noise filtering
38 Step of digital noise filtering
39 Conversion step
40 Normalization step
41 Storage step
42 Step of applying a decision algorithm
43 Step of measuring the increase in fluorescence

### DESCRIPTION OF EMBODIMENTS

With reference to the above figures, but not limited to, various embodiments of the invention are presented for better understanding.

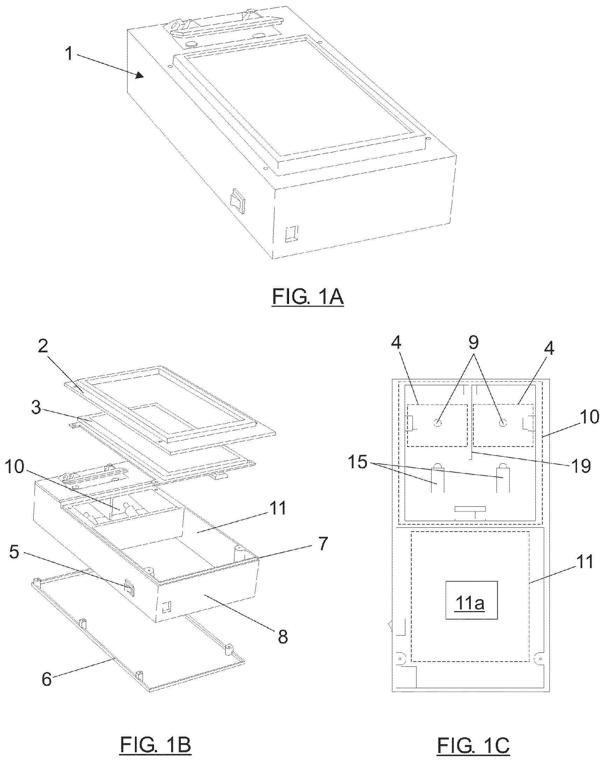

In **Fig. 1A** shows the external aspect of the device **1.** The size of the device **1** is reduced, e.g. by dimensions 125x253x50mm. **Figs. 1B-1C** show the internal structure of the device, which is portable, allowing rapid detection of industrial pathogens, and which can be used easily by nonspecialized personnel.

**Fig. 1B** shows an exploded view of the device 1. At the top of the device, the location of a top lid **2** and a touch-screen **3** for displaying and controlling, as a user interface, the operation and the result (for example, the model E349_Keyboard of the manufacturer Jun-Saxifragelec) can be seen. A first housing **10** for treating the samples and a second housing **11** mainly for the electronic part, an on/off switch **5** (e.g. model PRASA1-16F-BB000 from the manufacturer TE Connectivity / P&B) are also observed. A lower lid **6** and a frame **7** are illustrated that grant structural integrity to the device, and also a connection for electrical power **8** that serves for powering the device.

**Fig. 1C** shows the inside of the device. It is appreciated how the first housing **10** is intended for reactions and is divided into two individual compartments 4. In each compartment **4** there is a receptacle **9** for placing and fixing (for example, via insertion) a tube with a sample (not shown). Also in the first housing **10** there is a pair of LEDs **15** pointing towards the individual compartments **4.** The second housing **11** is mainly intended for electronic components, for example, a processor **11a.** These and other elements of the first reaction housing **10** are illustrated in greater detail in the following Fig.

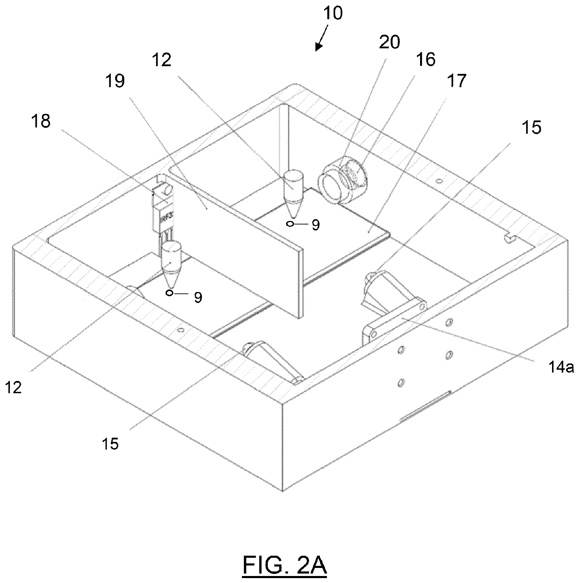

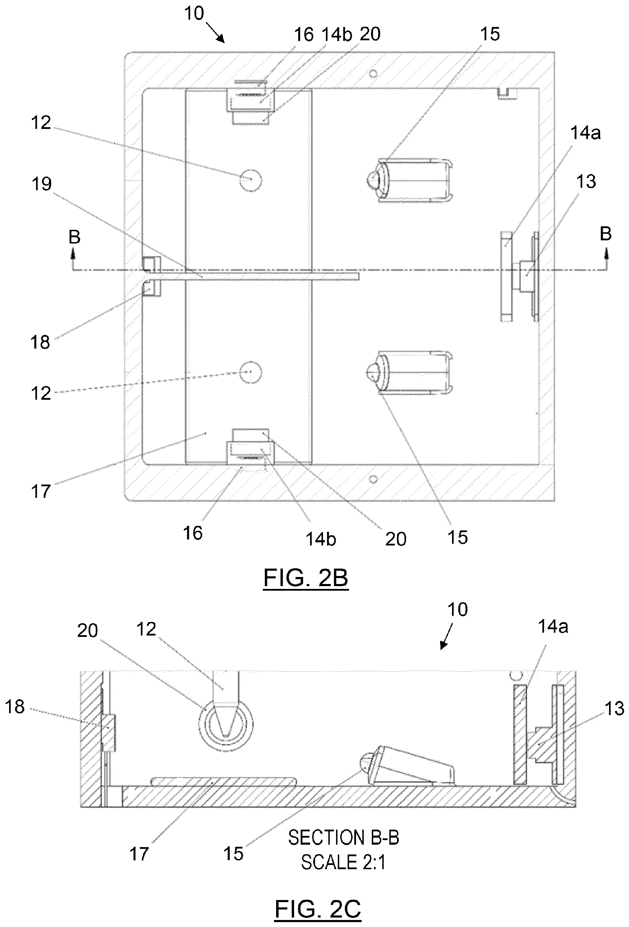

**FIGs. 2A-2C** are planes with more details of the first housing **10** for reactions. **Fig. 2A** is a perspective view. **Fig. 2B** is a plan view. **Fig. 2C** is a section on line B-B that has been enlarged. They are all described together. A reaction tube **12** is housed in a receptacle **9** where it is fitted and fixed. Each receptacle **9** is located at a certain distance from a photographic camera **13,** (for example, this camera may be the Raspberry Pi Camera 3099 model from the manufacturer Adafruit). The photographic camera **13** is preceded by an acrylic filter **14a** (e.g., PLEXIGLAS^{®} ORANGE 2C04 GT model). The acrylic filter **14a** allows to select wavelengths compatible with the detection mechanism. By replacing the characteristics of the filter, detection can be modified and thus adapted to different pathogen detection reactions and sample types. It is interesting to detect two different pathogens extracted from the same origin. Thus, the sample in each compartment **4** may be illuminated with a blue LED diode **15,** (e.g. Cree LED model C503B-BCN-CV0Z0462). A photodetector, in particular, a photodiode **16** generates an electrical (analog) signal proportional to the intensity of the detected optical signal (for example, a broad spectrum LED such as Hamamatsu Photonics model S1223-01). A preferably resistive, flexible, siliconetype heater **17** (e.g., RS Components model 245-528) is installed in the base of the housing **10.** Generally, it is sufficient to be able to increase the temperature of the sample in the range of 25 to 99°C, although it may vary according to the needs of each reaction. There is also in the first housing an amplifier **18** for amplifying the electrical signals, (e.g. a MOSFET transistor) a physical separating wall 19 between the sample compartments **4,** as well as lenses with protective covers **20.**

It should be mentioned that, the distribution of the components within the housing **10** for reactions, is designed in a way and manner that improves the detection of the optical signal to be studied. The location of the components takes into account as main contour conditions, the optimal operation of each element and of the set and that the device **1** is as compact as possible. In each compartment **4** is arranged the reaction tube **12** directly illuminated by its corresponding LED **15.** Perpendicular to the line connecting the LED **15** to the reaction tube **12** is the analog optical set formed by the protective lens cover **20,** an acrylic filter **14b** and the photodiode **16.** A wall **19** separates both compartments **4** from each other. As seen in the Figs., both sides of the housing **10** are symmetrical with respect to said wall **19.**

Various dimensions are provided in the planes for a prototype with the location of the reaction tube **12,** the photographic camera **13,** the acrylic filter **14,** the pair of LEDs **15,** the pair of photodiodes **16,** the heater **17,** the amplifier **18** contained in the housing **10.** The dimensions allow the correct operation of the aforementioned components, as well as the measures that relate the components to each other are shown in **Figs. 2A-2C****.** It should be understood that they are not unique, other measures would be possible. Other commercial components than those mentioned would also be applicable.

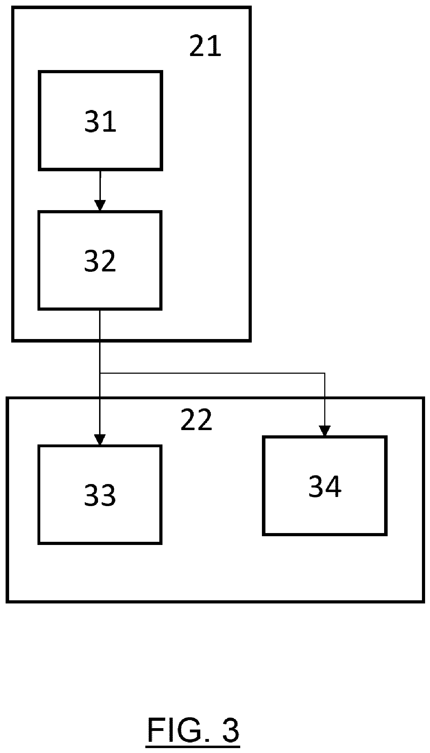

**Fig. 3** depicts in flowchart form several stages of the procedure **20** for detecting a pathogen relating to sample processing and assembly of the analysis reaction. In the method for detecting a pathogen there is a first sample pre-processing stage **21** in which the sample is prepared. The sample pre-processing stage **21** in turn includes several stages, a first mixing step **31** with a set of reagents and chemical solutions, specifically formulated to achieve the necessary transformations that allow the biological detection assay to be carried out. Thus, a second step of the processing stage **21** is a lysis step **32** for rupturing the membrane and/or cell wall of the microorganisms so that DNA or other genetic material is accessible in the medium.

A simple flow of operations for the lysis step **32** includes: transferring the sample by use of a disposable plastic pipette onto a sample extraction canister, containing a lysate solution, e.g., sodium hydroxide at a concentration of 100 mM.

Another alternative flow of operations for the lysis step **32** is focused on the treatment of poorly concentrated samples. To this end, lysis step **32** includes loading the sample into a syringe and filtering it with a filter of between 0.20 and 0.50 µm, for example, of the material polyvinylidene fluoride (PVDF), connecting a new pre-filled syringe with the lysis solution on that same filter, collecting the eluate in a canister with drop dispenser for the following operations. In both operation flows, the same procedure can be performed in parallel with a sterile control water sample, as a negative control.

In the procedure for detecting a pathogen there is a second reaction stage **22** including a step of placing the sample **33** already mixed with chemical and biological reagents necessary to carry out the biological detection. Similarly, step of placing a sterile substance **34** also mixed to establish a negative control. The basis of detection is an enzymatic nucleic acid amplification reaction, which may occur at constant temperature, e.g., recombinant polymerase amplification (RPA), or loop-mediated isothermal amplification; or with temperature cycles, such as polymerase chain reaction (PCR). In all these cases a fluorescent marker is included whose fluorescence emission intensity under a certain condition and wavelength will be proportional to the amount of pathogen in the sample. In order to carry out the specific detection of each pathogen it is necessary for design and manufacture of short sequences of DNA, between 20 and 40 base pairs, called oligonucleotides and/or probes. The correct design of these sequences is essential to carry out the detection of the pathogen correctly. These components will be added to the rest of the chemical reagents, resulting in a reaction mixture, to which the sample or negative control will be added.

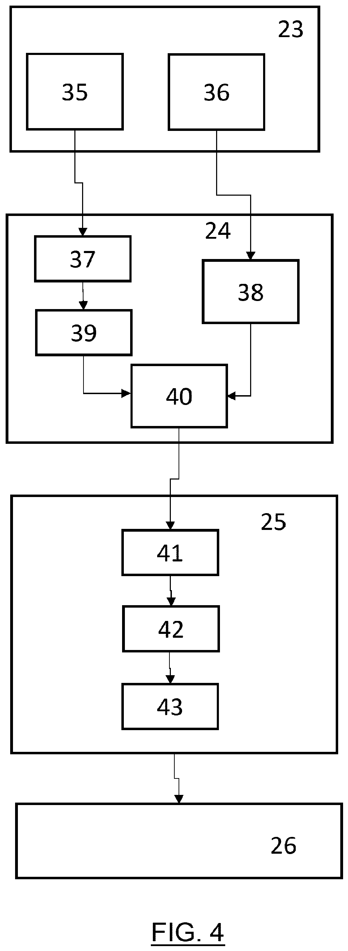

Next, in **Fig. 4** several process stages for detecting a pathogen are presented, continuation of the above. A third data acquisition stage **23** includes a step of collecting analog information **34** generated by the photodetector and a step of collecting digital information **35** generated by the camera, which can be done in parallel. The presence of the pathogen activates the reaction in a specific manner, the emission of an optical signal occurring in an intensity proportional to the amount of pathogen present in the sample. The device features a wavelength-determined illumination source, and two complementary detection mechanisms: photodetectors and a photographic camera.

The process continues with a fourth data pre-processing stage 24 which includes an analog noise filtering step **37** (in the photodiode voltage signal) and a digital noise filtering step **38** (in the image of the photographic camera). At a conversion step **39** the analog signal is digitalized. Following the data pre-processing stage **24,** a normalization step 40 is carried out and a moving average is applied to these numerical values of the pixels (digital information) and also to the voltage values (analog information) measured by the photodetectors. In a storage step **41** the results of such operations are stored in a temporary database, e.g. *InfluxDB.*

The method continues with a fifth prediction stage **25** where stored information of the data pre-processing stage 24 is collected from the database. The prediction stage **25** includes a step of applying a decision algorithm **42,** which may incorporate artificial intelligence models, for example, of the *machine learning* type, to improve prediction accuracy or sensitivity. If a negative control has been performed, there is a step of measuring the increase in fluorescence **43** produced in the sample during the experiment with respect to the negative control. Based on the growth value of said fluorescence level, it is determined whether the result of the sample is positive or negative. Additionally, the abundance of the pathogen detected can be determined from the signal intensity and, by comparison, from database of known values of the pathogen in question.

Finally, with a sixth visualization stage **26** the results of the detection by a user interface are shown.

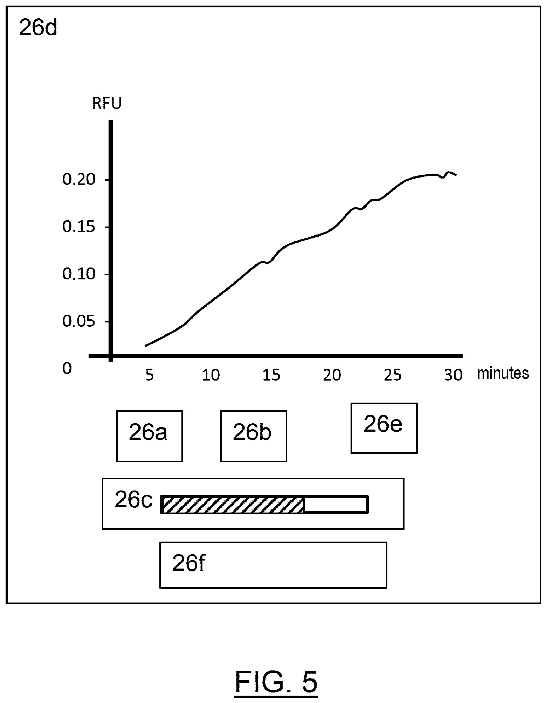

**Fig. 5** is an example of a graphical representation of the results of the visualization stage. They are displayed on a screen that may be touchable to act as a user interface and facilitate interaction with the device, allowing with a start/stop button **26a** to begin or end measurements of the device. The state **26b** in which the device is located is shown, which automatically regulates the temperature, and which indicates the following states: *heating* and ready. *Heating,* when the device is, automatically, regulating the temperature to reach the optimum reaction temperature. *Ready,* when the optimal temperature has been reached and the device may begin to take measurements. At the start of the measurement, the process progress bar **26c** is updated in real time and a display **26d** of the measurements obtained in the prediction stage is presented. The time in minutes is shown on the axis of abscissas, while the fluorescence intensity measured in relative fluorescence units (RFU) is shown on the coordinate axis. Once the experiment is completed, the generated data can be exported through the export button **26e** to generate the analysis of the historical series of results. Finally, the measurement result 26f is shown indicating whether or not the pathogen has been detected and, if applicable, a quantitative value proportional to the amount of pathogen detected.

Among the advantages of the embodiment of the invention, the following may be named: Portability: The device is easily transportable, does not require any specific condition in the place where it is to be used.

Operational autonomy: the device allows to complete the complete flow of analysis of industrial pathogens, including the concentration of the samples, if necessary.

Analysis rapidity: It allows to obtain reliable results in a few minutes.

Specificity: The use of genetic recognition techniques allows to unequivocally determine one or several specific pathogens in the sample, such as *Legionella sp., Listeria sp., Salmonella sp., E. coli, Clostridium sp., Campylobacter sp., Norovirus, Toxoplasma, Staphylococcus sp., Cronobacter sp., Vibrio sp.* or *Yersinia sp.,* among others.

Operational simplicity: It does not require the use of any type of specialized equipment or personnel to carry out the analyses.

Versatility: allows analyzing the presence of pathogens in a wide variety of samples, for example, water, surfaces or air

Quantitative analysis capacity: The equipment allows to quantify the abundance of a certain pathogen by comparison of the measured signal intensity with that of known values. Technological blending: The combination of software, hardware and biotechnological elements has a synergistic effect that improves the analytical properties of the device.

Digitization of data: The results of the analysis are obtained in digital format, so they can be easily shared and integrated into industrial facilities management platforms.

Modularity: slight changes in configuration allow the parallel analysis of several samples or different pathogens in the same sample, by selecting different optical lengths for each pathogen (e.g. two different bacteria).

The present invention is not to be construed as limited to the particular embodiments described herein, but is determined by the scope of the appended claims.

## Claims

1. Device (1) for detecting a pathogen in a sample (2) comprising:
- a first housing (10) for reactions comprising:
- an illumination source,
- two compartments (4), wherein each compartment (4) is designed to fix a reaction tube (12) in a position illuminated by the illumination source, wherein each compartment (4) comprises a photodetector configured to analogically detect a portion of light signal reflected by the reaction tube (12);
- a heater (17) configured to heat the reaction tubes (12),
- a photographic camera (13) configured to simultaneously capture a digital image of the two compartments (4), wherein the digital image includes the reaction tubes (12),
**characterized in that** the device (1) further comprises:
- a processing means configured to:
comparing the analog information obtained from the portion of light signal reflected of each reaction tube (12) with the digital information obtained from the image of each reaction tube (12), comparing the digital information and the analog information once digitalized, with an expected value corresponding to a specific pathogen and establishing the presence of the pathogen in the sample.

2. Device (1) according to claim 1, wherein the illumination source comprises two LEDs (15), wherein each LED (15) illuminates a compartment (4), wherein the compartments (4) are separated from each other by a wall (19), wherein the photodetector comprises a photodiode (16) and an associated filter (14b).

3. Device (1) according to claim 1 or 2, wherein the photographic camera (13) comprises a filter (14a) equal to the filter (14b) of the photodiode (16).

4. Device (1) according to any one of the preceding claims, wherein, in use, one of the reaction tubes (12) of one of the compartments (4) comprises a sterile substance, wherein the processing means are configured to perform a negative control by comparing both digital and analog information obtained from the reaction tube (12) containing the sample and the reaction tube (12) containing the sterile substance.

5. Device (1) according to any one of the preceding claims 1 to 3, wherein, in use, each reaction tube (12) of a compartment (4) comprises a sample with two pathogens to be detected.

6. Device (1) according to any one of the preceding claims, further comprising a user interface configured to display visual information related to detection in the sample from the analyzed images.

7. Device (1) according to any one of the preceding claims, wherein the filter (14b) associated with each photodiode (16) depends on the type of detection reaction of the pathogen.

8. Device (1) according to any one of the preceding claims, the detectable pathogen is:
- *Legionella sp.,*
- *Listeria sp.,*
- *Salmonella sp.,*
- *E. coli,*
- *Clostridium sp.,*
- *Campylobacter sp.,*
- *Norovirus,*
- *Toxoplasma,*
- *Staphylococcus sp.,*
- *Cronobacter sp.,*
- *Vibrio sp.,*
- *Yersinia sp.*
